(19)

Europäisches
Patentamt
European
Patent Office
Office européen
des brevets

(11) **EP 2 278 510 A1**

(12) **EUROPEAN PATENT APPLICATION**

(43) Date of publication:
**26.01.2011 Bulletin 2011/04**

(51) Int Cl.:
**G06F 19/00** *(2011.01)*

(21) Application number: **09305681.0**

(22) Date of filing: **16.07.2009**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(71) Applicant: **Statlife**
**94800 Villejuif (FR)**

(72) Inventor: **Ragusa, Stéphane**
**75007 Paris (FR)**

(74) Representative: **Blot, Philippe Robert Emile et al**
**Cabinet Lavoix**
**2, place d'Estienne d'Orves**
**75441 Paris Cedex 09 (FR)**

(54) **Method for determining a risk score**

(57) The present invention relates to a method for determining the risk that a multivariable-dependent event occurs for a first individual, based on at least two causative variables which values are determinable from the first individual, wherein the values of the at least two causative variables, and the occurrence, or not, of the event have been determined for individuals constituting a reference population, comprising:
(i) determining the values of the at least two causative variables from the first individual;

(ii) defining intervals respectively centred on the values of the at least two causative variables determined from the first individual;
(iii) determining the number of individuals of the reference population for which the event has occurred and for which the values of each of the at least two causative variables are comprised in the intervals defined in (ii);
(iv) determining therefrom the risk of occurrence of the event for the first individual.

EP 2 278 510 A1

**Description**

Field of the invention

The present invention relates to a method for determining a risk of occurrence of an event, in particular a pathological event, for an individual.

Technical background

The importance of methods for accurately predicting the risk of occurrence of an event for an individual is particularly marked in the field of human health, either for research or clinical purpose.

Indeed, determining that an individual is at risk of a particular adverse event allows preventive measures to be taken, such as prophylactic pharmaceutical treatments. Besides, a precise determination of the risk, usually in the form of a risk score, is also useful where a treatment is likely to induce side effects, since it allows determining the benefit/risk ratio of the intended treatment.

Methods for determining risk scores are usually set up from the study of a reference population for which several variables are determined (e.g. age, number of relatives in whom the pathological event has occurred, levels of certain biochemical markers) in addition to the recording of the occurrence or not of the event. In most cases several causative variables, or risk factors, are identified, and a multiparametric mathematical model is derived therefrom.

Such methods are particularly useful for predicting a cardiovascular risk and the risk of occurrence of breast cancer.

In this regard, one of the most used methods is the Gail model for the prediction of breast cancer (Gail et al. (1989) J. Natl. Cancer Inst. 81:1879-1886). Thus, a 5-year risk of breast cancer higher than 1.66% calculated with this model is usually considered justifying the prescription of a breast cancer preventive treatment, in particular with tamoxifen.

The Gail model has been developed using data from a nested case-control subset of the 284,780 women participating in the Breast Cancer Detection Demonstration Project (BCDDP). The BCDDP women were predominantly Caucasian US citizens, 35-79 years of age, and receiving annual mammogram screening. The Gail model defines the log odds of developing breast cancer as:

$$\text{Log odds} = -0,74948 + 0,09401 \times \text{age at menarche} + 0,52926 \times \text{number of biopsies} + 0,21863 \times \text{age at first live birth} + 0,95830 \times \text{number of relatives with breast cancer} + 0,01081 \times \text{age} (<50 \textit{ versus } 50+) - 0,28804 \times (\text{number of biopsies} \times \text{age}) - 0,19081 \times (\text{age at first birth} \times \text{number of relatives with breast cancer}).$$

Such methods however suffer from many drawbacks, in particular where they are applied to individuals differing from the reference population or where additional causative variables are to be added to the model.

Thus, the Gail model for instance, cannot be directly applied to non-Caucasian and/or non-American individuals, as is notably illustrated by Gail et al. (2007) J. Natl. Cancer Inst. 99:1782-1992, in which completely different coefficients are defined for the predictive model to be applied to African-American women, thereby evidencing the great influence of genetic background and/or environmental conditions in risk assessment. In addition, when confronted with the task of taking integrating mammography density as a new causative variable in the Gail model, Chen (2006) J. Natl. Cancer Inst. 98:1215-1226 in effect developed a new model.

Accordingly, there is a need for a method for determining a risk of occurrence of an event for an individual which could yield accurate results, even when the individual is different from the reference population used by the method. Besides, there is also a need for more flexible methods liable to easily integrate additional causative variables.

Summary of the invention

The present invention solves these problems by providing a method for determining the risk that a multivariable-dependent event occurs for a first individual, based on at least two causative variables which values are determinable from the first individual, wherein the values of the at least two causative variables, and the occurrence, or not, of the event have been determined for individuals constituting a reference population, comprising:

(i) determining the values of the at least two causative variables from the first individual;

(ii) defining intervals respectively centred on the values of the at least two causative variables determined from the first individual;

(iii) determining the number of individuals of the reference population for which the event has occurred and for which the values of each of the at least two causative variables are comprised in the intervals defined in (ii);

(iv) determining therefrom the risk of occurrence of the event for the first individual.

The method of the invention is advantageous in that the risk of occurrence of the event for the individual is determined from individuals of the reference population which are the closest neighbours of the individual, as regards the values of the causative variables. Accordingly, the method of the invention does not require that coefficients to be applied to the causative variables are obtained from the whole reference population in order to determine the risk of occurrence of the event. Besides, the method of the invention can therefore yield accurate results, even where a characteristic of the individual can only be determined from a subgroup of individuals of the reference population (e.g. ethnicity or country of residence). In addition, the method of the invention can readily accommodate changes, such as addition or suppression, of causative variables. The method of the invention can thus be carried out with a varying number of causative variables, depending on the causative variables for which values are available for the individual or for the reference population.

Detailed description of the invention

As intended herein, the expression "multivariable-dependent event" relates to an event which probability of occurrence for an individual can be influenced by at least two causative variables.

Preferably, the "individual" as defined above is a human. Preferably also and the "event" as defined above is a pathological event. The pathological event can be of any type, such as death, or the occurrence of a disease, such as cancer, in particular breast cancer or prostate cancer, a cardiovascular disease, or a neurodegenerative disease, such as Alzheimer disease. The pathological event can also be a side effect arising from a preventive or therapeutical treatment of the individual, such as breast cancer, which risk of occurrence can be increased in the frame of substitutive hormonal treatment of menopause.

The expression "causative variable" relates to any risk factor liable to have an influence on the probability of occurrence of the event. The causative variable can be of a qualitative nature or of a quantitative value. Exemplary qualitative causative variables can be ethnicity, current hormone therapy use, etc. Exemplary quantitative variables, include age, body mass index, or the level of biochemical markers, such as Prostate-Specific Antigen. By way of example, causative variables for breast cancer are given in Barlow et al. (2006) J. Natl. Cancer Inst. 98:1204-1214.

The "reference population" can be of any type, however it is preferred that it comprises at least 1000 individuals.

In a preferred embodiment of the invention, the length of the respective intervals varies inversely with the increase in risk associated to the respective causative variables in the occurrence of the event.

The expression "increase in risk" relates to an estimation of the increased risk of occurrence of the event for the individual in respect to a single causative variable. Numerous methods are known in the art for determining the above-defined increase in risk. In particular, the increase in risk can be a relative risk (RR) minus 1 (RR - 1), or an odds ratio (OR) minus 1 (OR - 1).

One of skill in the art can readily determine a "relative risk" or an "odds ratio".

Generally, a relative risk is defined as the ratio of (i) the percentage of individuals of a population in whom the event has occurred having certain values of the causative variable to (ii) the percentage of individuals of the same population in whom the event has occurred having the complementary values to that defined in (i) of the causative variable.

Generally also, the odds ratio is defined as the ratio of (i) the ratio of the number of individuals in whom the event has occurred to the number of individuals in whom the event has not occurred, wherein the individuals have certain values of the causative variable, to (ii) the ratio of the number of individuals in whom the event has occurred to the number of individuals in whom the event has not occurred, wherein the individuals have the complementary values to that defined in (i) of the causative variable.

The increase in risk can also be standardized with respect to the variations of the values of the causative variable. In particular, the increase in risk can be expressed as an increase of the risk in respect to an elementary variation (*i.e.* increase or decrease) of the values of the causative variables.

As will be clear to one of skill in the art, the increase in risk can be an average increase in risk. Determining an "average increase in risk" is useful where the increase in risk varies as a function of values of its causative variable and where the causative variable can have more than two values. In particular, the average increase in risk can be calculated by averaging individual increases in risk, such as OR - 1 or RR - 1, optionally standardized with respect to the variations of the causative variable.

It should also be clear to one of skill in the art that approximated values can be used for the increase in risk.

In particular, only an order in size of the increase in risk can be used within the frame of the invention.

In another preferred embodiment of the invention, the length of the intervals varies with the standard deviation from the mean (SD) of the values determined for the respective causative variables in the population. Here also, it should be clear to one of skill in the art that approximated values of SD, in particular approximated values giving only an order in size of SD, can be used within the frame of the invention.

Accordingly, in a particularly preferred embodiment of the invention, the length of each interval is proportional to the ratio of:

a) the standard deviation from the mean for the corresponding causative variable, to
b) the increase in risk, as defined above, associated to the corresponding causative variable in the occurrence of the event,
and wherein the intervals share a same coefficient of proportionality.

These embodiments are advantageous in that they can increase the accuracy of the method by ensuring that only the closest neighbours are retained where the causative variable has a strong influence on the risk of occurrence of the even, whereas farther neighbours can be retained when the influence of the causative variable is less important.

Preferably also, the length of the intervals is such that a predetermined number of individuals of the reference population have values of each of the at least two causative variables comprised in the intervals. In this case, it is preferred that at least 100 or 1000 individuals have values of each of the at least two causative variables comprised in the intervals. Besides, it is also preferred that the length of the intervals is such that a predetermined number of individuals of the reference population for which the event has occurred have values of each of the at least two causative variables comprised in the intervals. In this case, it is preferred that at least 30 individuals, more preferably at least 100 individuals, have values of each of the at least two causative variables comprised in the intervals.

In a preferred embodiment of the invention, step (iv) is carried by dividing the number determined in (iii) by the number of individuals of the reference population for which the values of each of the at least two causative variables are comprised in the intervals. In another preferred embodiment of the invention, step (iv) is carried by dividing the number determined in (iii) by the total number of individuals of the reference population.

In another embodiment of the invention, the above-defined method comprises:

- determining a first risk of occurrence of a pathological event for the individual based on a first reference population, wherein the individuals of the first reference population have received no treatment intended to reduce the occurrence of the pathological event;
- determining a second risk of occurrence of a pathological event of the individual based on a second reference population, wherein the individuals of the second reference population have received a treatment intended to reduce the occurrence of the pathological event, the values of the at least two causative variables have been determined before the individuals of the second population have received the treatment, and the occurrence, or not, of a pathological event has been determined after the individuals of the second reference population have received the treatment;
- comparing the values determined for the first and second risks;
- determining that the treatment is suitable to reduce the risk of occurrence of the pathological event if the value of the second risk is significantly lower than the value of the first risk.

This embodiment is advantageous, in that it allows determining the benefit, *i.e.* the reduction in the risk of occurrence of the pathological event, that an individual may expect from a treatment.

In yet another embodiment of the invention, the above-defined method comprises:

- determining a first risk of occurrence of a pathological event of the individual based on a first reference population, wherein the individuals of the first reference population have received a treatment liable to induce the occurrence of the pathological event, the values of the at least two causative variables have been determined before the individuals of the first population have received the treatment, and the occurrence, or not, of a pathological event has been determined after the individuals of the first reference population have received the treatment;
- determining a second risk of occurrence of a pathological event for the individual based on a second reference population, wherein the individuals of the second reference population have not received the treatment;
- comparing the values determined for the first and second risks;
- determining that the treatment is suitable for the individual if the value of the first risk is not significantly higher than the second risk.

This embodiment is advantageous in that it allows determining the risk associated to a treatment by determining the risk of occurrence of a known side effect of the treatment (the pathological event).

In a further embodiment, the method of the invention comprises:

- determining a first risk of occurrence of a first pathological event for the individual based on a first reference population, wherein the individuals of the first reference population have received no treatment intended to reduce the occurrence of the pathological event;
- determining a second risk of occurrence of a first pathological event of the individual based on a second reference population, wherein the individuals of the second reference population have received a treatment intended to reduce the occurrence of the first pathological event, the values of at least two causative variables have been determined before the individuals of the second population have received the treatment, and the occurrence, or not, of a first pathological event has been determined after the individuals of the second reference population have received the treatment;
- determining a first risk of occurrence of a second pathological event of the individual based on the first reference population, wherein the second pathological event is liable to arise in response to said treatment;
- determining a second risk of occurrence of the second pathological event of the individual based on the second reference population, wherein the values of at least two causative variables have been determined before the individuals of the second population have received the treatment, and the occurrence, or not, of the second pathological event has been determined after the individuals of the second reference population have received the treatment;
- comparing the values determined for the first and second risks of occurrence of the first and second pathological event;
- determining therefrom if the treatment is suitable for the individual.

This embodiment combines the advantages of the two previous embodiments by enabling to determine the benefit/risk ratio of a treatment. Depending on the determined risks if occurrence of the first and second pathological events and on the respective seriousness of the first and second pathological events, one of skill in the art can reach a decision as to the opportunity of undertaking the treatment or give the individual the necessary information for him to give an informed consent before undertaking the treatment.

For instance, where substitutive hormonal or Selective Estrogen Receptor Modulators (SERMs) treatment for a post-menopausal woman (the individual) is considered, this embodiment enables determining the benefit that the individual may expect from the treatment, for instance in terms of a reduced risk of occurrence of osteoporosis (the first pathological event), as well as the risk that the individual may face when undertaking the treatment, such as the risk of occurrence of breast cancer (the second pathological event).

The invention will be further explained by the following non-limiting example.

Example

Determination of the risk of occurrence of breast cancer in an individual

Causative variables for breast cancer are notably described in Barlow et al. (2006) J. Natl. Cancer Inst. 98: 1204-1214. In the frame of the present Example, age, body mass index (BMI), and breast density will be considered.

The individual whose risk is to be determined is a woman aged 60, with a BMI of 25, and a breast density of 2.

The reference population is as defined in the following table:

| Causative variable | Mean | Standard Deviation (SD) | Average Increase in Risk |
|---|---|---|---|
| Age (years) | 62 | 10 | 0.1 per increase of 1 year |
| BMI ($kg/m^2$) | 28 | 3 | 0.05 per increase of 1 $kg/m^2$ |
| Breast density (BI-RAD) | 2.3 | 0.5 | 1 per increase of 1 BI-RAD |

In the present case the above average increases in risk are determined from the odds ratios given for the respective causative variable in Table 5 of Barlow *et al.* (2006) *J. Natl. Cancer Inst.* **98**:1204-1214 as indicated below.

For age, the following odds ratios are available:

| Age (in years) | Odds ratio | Increase in risk[*] |
|---|---|---|
| 45-49 | 1 (reference) | - |

(continued)

| Age (in years) | Odds ratio | Increase in risk* |
|---|---|---|
| 50-54 | 1.33 | (1.33 - 1) / 5 = 0.07 |
| 55-59 | 1.96 | (1.96 - 1) / 10 = 0.1 |
| 60-64 | 2.27 | (2.27 - 1) / 15 = 0.08 |
| 65-69 | 2.47 | (2.47 - 1) / 20 = 0.07 |
| 70-74 | 2.79 | (2.79 - 1) / 25 = 0.07 |
| 75-79 | 3.03 | (3.03 - 1) / 30 = 0.07 |
| 80-84 | 3.33 | (3.33 - 1) / 35 = 0.07 |
| *The increase in risk for age as causative variable is defined as the additional risk in respect to the reference, for an increase of 1 year. The number of years separating the reference interval and another interval is obtained by subtracting the mean age of the reference interval to the mean age of the other interval. | | |

The average increase in risk is then obtained by averaging the determined increases in risk, which yields 0.075, and is approximated to 0.1.

For BMI, the following odds ratios are available:

| BMI (in kg/m$^2$) | Odds ratio | Increase in risk* |
|---|---|---|
| <25 | 1 (reference) | - |
| 25-29.99 | 1.14 | (1.14 - 1) / 2.5 = 0.06 |
| 30-34.99 | 1.28 | (1.28 - 1) / 7.5 = 0.04 |
| ≥35 | 1.47 | (1.47 - 1) / 10 = 0.05 |
| *The increase in risk for BMI as causative variable is defined as the additional risk in respect to the reference, for an increase of 1 kg/m$^2$. The number of kg/m$^2$ separating the reference interval and another interval is obtained by subtracting 25 to the mean of the other interval. For the ≥35 interval, a difference of 10 kg/m$^2$ is used. | | |

The average increase in risk is then obtained by averaging the determined increases in risk, which yields 0.05.

For breast density the following odds ratios are available:

| Breast density (in BI-RADs) | Odds ratio | Increase in risk* |
|---|---|---|
| 1 | 1 (reference) | - |
| 2 | 2.09 | (2.09 - 1)/ 1 = 1.09 |
| 3 | 2.95 | (2.95 - 1)/ 2 =0.97 |
| 4 | 3.15 | 3.15-1/3=0.72 |
| *The increase in risk for breast density as causative variable is defined as the additional risk in respect to the reference, for an increase of 1 BI-RAD. The number of BI-RADs separating the reference interval and another interval is obtained by subtracting 25 to the mean of the other interval | | |

The average increase in risk is then obtained by averaging the determined increases in risk, which yields 0.93, and is approximated to 1.

The intervals centred on the values of the causative variables are then as follows:

| Causative variable | Intervals |
| --- | --- |
| Age (years) | $60 \pm 10 / 0.1 \times \lambda$ |
| BMI ($kg/m^2$) | $25 \pm 3 \times 0.05 \times \lambda$ |
| Breast density (BI-RAD) | $2 \pm 0,5 / 1 \times \lambda$ |
| $\lambda$ can either be chosen so that a predetermined number of individuals of the reference population have values of the causative variables comprised in the above intervals (e.g. 100 or 1000) or so that a predetermined number of individuals of the reference population who have experienced breast cancer have values of the causative variables comprised in the above intervals (e.g. 30). | |

The risk of occurrence of breast cancer of the individual can then be determined by dividing the number of individuals of the reference population in whom breast cancer has occurred and whose values of the causative variables are comprised within the above intervals, by the total number of individuals of the reference population whose values of the causative variables are comprised within the above intervals.

**Claims**

1. A method for determining the risk that a multivariable-dependent event occurs for a first individual, based on at least two causative variables which values are determinable from the first individual, wherein the values of the at least two causative variables, and the occurrence, or not, of the event have been determined for individuals constituting a reference population, comprising:

   (i) determining the values of the at least two causative variables from the first individual;
   (ii) defining intervals respectively centred on the values of the at least two causative variables determined from the first individual;
   (iii) determining the number of individuals of the reference population for which the event has occurred and for which the values of each of the at least two causative variables are comprised in the intervals defined in (ii);
   (iv) determining therefrom the risk of occurrence of the event for the first individual.

2. The method according to claim 1, wherein the length of the respective intervals varies inversely with the increase in risk associated to the respective causative variables in the occurrence of the event.

3. The method according to claim 1 or 2, wherein the length of the intervals varies with the standard deviation from the mean (SD) of the values determined for the respective causative variables in the population.

4. The method according to any of claims 1 to 3, wherein the length of each interval is proportional to the ratio of:

   a) the standard deviation from the mean for the corresponding causative variable, to
   b) the increase in risk associated to the corresponding causative variable in the occurrence of the event,
   and wherein the intervals share a same coefficient of proportionality.

5. The method according to any of claims 1 to 4, wherein the length of the intervals is such that a predetermined number of individuals of the reference population have values of each of the at least two causative variables comprised in the intervals.

6. The method according to any of claims 1 to 5, wherein the length of the intervals is such that a predetermined number of individuals of the reference population for which the event has occurred have values of each of the at least two causative variables comprised in the intervals.

7. The method according to any of claims 1 to 6, wherein step (iv) is carried by dividing the number determined in (iii) by the number of individuals of the reference population for which the values of each of the at least two causative variables are comprised in the intervals.

8. The method according to any of claims 1 to 7, wherein step (iv) is carried by dividing the number determined in (iii) by the total number of individuals of the reference population.

9. The method according to any of claims 1 to 8, wherein the individual is a human and the event is a pathological event.

10. The method according to claim 9, comprising:

   - determining a first risk of occurrence of a pathological event for the individual based on a first reference population, wherein the individuals of the first reference population have received no treatment intended to reduce the occurrence of the pathological event;
   - determining a second risk of occurrence of a pathological event of the individual based on a second reference population, wherein the individuals of the second reference population have received a treatment intended to reduce the occurrence of the pathological event, the values of the at least two causative variables have been determined before the individuals of the second population have received the treatment, and the occurrence, or not, of a pathological event has been determined after the individuals of the second reference population have received the treatment;
   - comparing the values determined for the first and second risks;
   - determining that the treatment is suitable to reduce the risk of occurrence of the pathological event if the value of the second risk is significantly lower than the value of the first risk.

11. The method according to claim 9, comprising:

   - determining a first risk of occurrence of a pathological event of the individual based on a first reference population, wherein the individuals of the first reference population have received a treatment liable to induce the occurrence of the pathological event, the values of the at least two causative variables have been determined before the individuals of the first population have received the treatment, and the occurrence, or not, of a pathological event has been determined after the individuals of the first reference population have received the treatment;
   - determining a second risk of occurrence of a pathological event for the individual based on a second reference population, wherein the individuals of the second reference population have not received the treatment;
   - comparing the values determined for the first and second risks;
   - determining that the treatment is suitable for the individual if the value of the first risk is not significantly higher than the second risk.

12. The method according to claim 9, comprising:

   - determining a first risk of occurrence of a first pathological event for the individual based on a first reference population, wherein the individuals of the first reference population have received no treatment intended to reduce the occurrence of the pathological event;
   - determining a second risk of occurrence of a first pathological event of the individual based on a second reference population, wherein the individuals of the second reference population have received a treatment intended to reduce the occurrence of the first pathological event, the values of at least two causative variables have been determined before the individuals of the second population have received the treatment, and the occurrence, or not, of a first pathological event has been determined after the individuals of the second reference population have received the treatment;
   - determining a first risk of occurrence of a second pathological event of the individual based on the first reference population, wherein the second pathological event is liable to arise in response to said treatment;
   - determining a second risk of occurrence of the second pathological event of the individual based on the second reference population, wherein the occurrence, or not, of the second pathological event has been determined after the individuals of the second reference population have received the treatment;

- comparing the values determined for the first and second risks of occurrence of the first and second pathological event;
- determining therefrom if the treatment is suitable for the individual.

Europäisches
Patentamt

European
Patent Office

Office européen
des brevets

## EUROPEAN SEARCH REPORT

Application Number

EP 09 30 5681

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 808 790 A (STATLIFE [FR])<br>18 July 2007 (2007-07-18)<br>* the whole document * | 1-12 | INV.<br>G06F19/00 |
| X | WO 2008/152212 A (STATLIFE [FR]; RAGUSA STEPHANE [FR])<br>18 December 2008 (2008-12-18)<br>* the whole document * | 1-12 | |
| X | WO 2008/085308 A (CARDIAC PACEMAKERS INC [US]; CAZARES SHELLEY M [US]; PETERSON JON [US]) 17 July 2008 (2008-07-17)<br>* the whole document * | 1-12 | |
| A | EP 1 244 037 A (GE FRANKONA MAN SERVICE GMBH [DE]) 25 September 2002 (2002-09-25)<br>* the whole document * | 1 | |
| A | WO 2009/069152 A (DECODE GENETICS EHF [IS]; GUDMUNDSSON JULIUS [IS]; SULEM PATRICK [IS];) 4 June 2009 (2009-06-04)<br>* the whole document * | 1 | TECHNICAL FIELDS SEARCHED (IPC)<br><br>G06F |

The present search report has been drawn up for all claims

| Place of search | Date of completion of the search | Examiner |
|---|---|---|
| The Hague | 6 October 2009 | Itoafa, Alex |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 09 30 5681

This annex lists the patent family members relating to the patent documents cited in the above-mentioned European search report.
The members are as contained in the European Patent Office EDP file on
The European Patent Office is in no way liable for these particulars which are merely given for the purpose of information.

06-10-2009

| Patent document cited in search report | | Publication date | Patent family member(s) | Publication date |
|---|---|---|---|---|
| EP 1808790 | A | 18-07-2007 | NONE | |
| WO 2008152212 | A | 18-12-2008 | NONE | |
| WO 2008085308 | A | 17-07-2008 | AU 2007342523 A1<br>EP 2096993 A1<br>US 2008162182 A1 | 17-07-2008<br>09-09-2009<br>03-07-2008 |
| EP 1244037 | A | 25-09-2002 | NONE | |
| WO 2009069152 | A | 04-06-2009 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **Gail et al.** *J. Natl. Cancer Inst.,* 1989, vol. 81, 1879-1886
- **Gail et al.** *J. Natl. Cancer Inst.,* 2007, vol. 99, 1782-1992
- **Chen.** *J. Natl. Cancer Inst.,* 2006, vol. 98, 1215-1226
- **Barlow et al.** *J. Natl. Cancer Inst.,* 2006, vol. 98, 1204-1214